# EUROPEAN PATENT APPLICATION

(11) **EP 4 563 528 A1**
(43) Date of publication of application: **04.06.2025**
(21) Application number: 23845756.8
(22) Date of filing: 13.07.2023
(51) Int. Cl.: C01B 33/12, A61K 9/51, B82Y 5/00

(54) **SILICA MESOPOROUS NANOPARTICLES AND USE THEREOF FOR CAPTURING IMMUNOGLOBULINS**

(30) Priority: 29.07.2022 ES 202230701
(71) Applicant: Servicio Andaluz de Salud, 41092 Sevilla (ES); Universidad De Malaga, E-29071 Málaga (ES)
(72) Inventor: PARIS FERNANDEZ DE LA PUENTE, Juan Luis, 29590 Málaga (ES); FERNÁNDEZ DUARTE, Tahia Diana, 29590 Málaga (ES); MONTAÑEZ VEGA, María Isabel, 29590 Málaga (ES); MAYORGA MAYORGA, Cristobalina, 29590 Málaga (ES); TORRES JAÉN, María José, 29590 Málaga (ES)
(74) Representative: Pons
(86) International application number: PCT/ES2023/070450
(87) International publication number: WO 2024/023382

(57) **Abstract**

The present invention relates to a silica mesoporous nanoparticle comprising a covalently bound protein G' or protein A, the composition comprising said nanoparticle, the use thereof for capturing, purifying, eliminating and/or isolating immunoglobulins, preferably IgG, as well as a method for purifying an immunoglobulin, methods for pre-treating samples in order to subsequently diagnose allergies, infections and/or autoimmune diseases in a patient, and said diagnostic methods.

## Description

The present invention relates to silica mesoporous nanoparticles comprising a protein G' or protein A coupled on their surface and use thereof for capturing immunoglobulins, being useful for purifying them from samples and for pre-treating biological samples, wherein purifying, concentrating and/or eliminating certain immunoglobulins is desirable such as, for example, but not limited to, for diagnosing allergies. Therefore, the present invention is encompassed within clinical practice, preferably for pre-treating biological samples obtained from subjects to facilitate subsequently diagnosing pathological conditions in which there is a high expression of immunoglobulins.

### BACKGROUND OF THE INVENTION

The *in vitro* detection of immunoglobulins in serum is a widely used strategy for the diagnosis of a number of pathologies, from infectious diseases to autoimmune and allergic diseases. The general aim of these techniques is the detection of some type of antibody specific to a given antigen, which may be present in an infectious agent, in the patient's own body (self-antigen) or come from other exogenous sources (such as allergens).

Most *in vitro* allergy diagnostic techniques are based on detecting allergen-specific immunoglobulin E (IgE). However, for certain types of allergy, the amount of IgE present in serum is very low and may fall below the detection limits of currently available techniques due to their low sensitivity. This requires the use of *in vivo* diagnostic methods (controlled allergen exposure skin tests or challenge skin tests), which not only entail a higher cost for the health system, but pose a risk to the patient, especially in cases of severe allergic reactions in which exposure to the allergen during the diagnostic test may trigger an anaphylactic reaction.

Hence the great usefulness of the *in vitro* diagnosis of allergies, specifically immunoassays. These consist of a solid phase to which an allergenic structure that is incubated with the serum from patients suspected of having allergies is conjugated. Subsequently, the use of a secondary anti-lgE developer antibody (with a radioactive, fluorescent or enzyme labelling that participates in a colorimetric reaction) allows the identification of the presence of IgE specific to the tested allergen in the patient's serum. In this context, there are several factors that limit the sensitivity of these diagnostic tests, but one of the main factors derives from the high concentration of IgG in serum compared to the amount of IgE. The presence of allergen-specific IgG occupies part of the allergenic structures present in the solid phase, which reduces or blocks the recognition sites available for the binding of specific IgE, which is the analyte to be detected. It is not only the binding of allergen-specific IgG that limits sensitivity, but rather the very presence of high amounts of IgG, even if it is not specific to the allergen, also limits the sensitivity of *in vitro* diagnostic testing for allergies. Additionally, the widespread use of multiplex allergy diagnostic methods (simultaneous detection of immunoglobulins against multiple allergens) worsens this situation, as false positives have been described due to incorrect detection of allergen-specific IgG (of unknown clinical usefulness) as if it were allergen-specific IgE (which is the validated biomarker for diagnosis). As a result, the use of systems for pre-treating a sample based on the removal of IgG from serum prior to its analysis by *in vitro* diagnostic testing for allergies has been proposed. In order to carry out this removal, commercial systems for capture based on particles or columns conjugated to a protein with selective human IgG constant region binding capacity (and with no IgE binding capacity), such as protein A and protein G, have been used. There are multiple commercial systems in which one of these two proteins (or a combination of both) has been bound to the surface of particles or to the solid phase of columns. Therefore, these materials can be used for purifying or removing IgG from a fluid. However, these systems have different limitations: they require complex protocols and high incubation times or sample volumes and have a low IgG capture capacity (up to 6-125 µg IgG/mg material), which requires multiple cycles of serum treatment to achieve the required level of capture. These requirements have prevented these strategies from being transferred to clinical practice, and there is currently no commercial system that has been validated for use on samples for diagnostic purposes.

All commercially available products consist of a solid phase (in the form of nano- or microparticles in powder or suspension or in an affinity column) with protein A, protein G or a combination of both conjugated to the surface of the material. Although there are products made with particles of different compositions and sizes, there are no commercial systems for removing IgG based on mesoporous particles with pores in the range of 2-50 nm. In this sense, Hu *et al.* (J. Hu, et al., Microporous Mesoporous Mater. 2014, 197, 180-184) described the preparation of magnetic particles coated with mesoporous silica to which protein G was covalently bound. The starting material used had a pore size centred on 3.27 nm, and from this, an expanded pore material (with a very wide distribution of pore diameters between 5 and 100 nm) was also obtained. The particles used in this study exhibited a diameter in the range of 50 - 100 nm, with a relatively broad size distribution (estimated from the transmission electron microscopy (TEM) images shown in the paper). In this study, a higher IgG capture capacity was observed in the expanded pore material, although the difference was modest (51 µg IgG/mg of expanded pore material versus 41 µg IgG/mg of 3.27 nm pore material). Additionally, the IgG capture capacity they showed (51 µg IgG/mg nanoparticles) is still too low for use in clinical practice. Furthermore, the pore size distribution in this material is very broad and TEM images show very little homogeneity in the porosity of the particles. Moreover, the surface area of this expanded pore material (43.4 m²/g) is very small compared to other mesoporous materials, which may limit the amount of bound protein and, therefore, IgG capture capacity. These two limitations derive from the method used to produce the materials, based on obtaining materials with smaller pore sizes, which are subsequently expanded, which gives rise to a large heterogeneity in the final pore size obtained and a large decrease in the surface area of the material.

Therefore, there is a need in the state of the art to provide materials based on obtaining mesoporous particles from which improved immunoglobulin capture results can be obtained, which are also reliable and reproducible and allow being transferred to clinical practice. Said materials should furthermore exhibit a highly homogeneous particle size and pore size distribution, allowing the reproducibility thereof and maximising the efficiency of immunoglobulin capture.

### DESCRIPTION OF THE INVENTION

The inventors have observed that the immunoglobulin capture by a mesoporous material with a given pore size coupled to Protein G' (protein G devoid of the albumin recognition region) or to Protein A is much higher than when a material with a pore size outside the mesoporosity ranges described herein is used. On the basis of this observation, the inventors designed a nanoparticle exhibiting a highly homogeneous particle size and pore size distribution compared to other mesoporous nanoparticles in the state of the art, which allows for the immunoglobulin, particularly IgG, capture more effectively than with other existing methodologies.

The novelty of the present invention lies in the fact that by binding protein G' or protein A to mesoporous nanoparticles having the pore size indicated herein (greater than 10 nm in diameter, preferably between 10 nm and 15 nm in diameter, more preferably 12 nm in diameter), immunoglobulin capture capacity is significantly increased. In the examples shown below, it can be seen how other pore sizes tested by the inventors for silica mesoporous nanoparticles, such as 5.75 nm or 8.53 nm, resulted in a lower percentage of protein G' bound to the nanoparticle (6.88% and 7.89%, respectively), compared to 8.94% obtained with the nanoparticles of the invention (see Table 2 of the Examples shown below).

It is important to note that as a consequence of the previous paragraph, the IgG capture capacity obtained with the nanoparticles of the invention (450-800 µg of IgG per mg of nanoparticles) is surprisingly much higher than that shown by other commercial systems or systems described in research (see Table 3 of the Examples), representing at least a 4-fold improvement with respect to the best performing commercial product. Therefore, it is demonstrated that, compared to commercially available products and to the other types of silica mesoporous nanoparticles with different pore sizes prepared in the Examples of the present invention, nanoparticles with larger pore size (nanoparticles of the invention) exhibited a significantly higher IgG capture capacity than the other systems, definitively confirming that by means of controlling the pore size of the nanoparticles to which protein G' is bound, its IgG removal capacity can be optimised.

This increase in the efficacy for capturing immunoglobulins is useful, for example, for pre-treating samples in order to subsequently diagnose allergies where IgE is at very low concentrations in blood/serum, so the presence of IgG at high concentrations may mask or hinder the detection of IgE, giving way to false negatives in the diagnosis of an allergic process. Therefore, the inventors also propose, based on the nanoparticles of the invention described herein, a method for pre-treating samples and diagnosing allergies that overcomes the drawbacks of other diagnostic techniques disclosed in the state of the art.

Another advantage derived from the present invention is that once the nanoparticles with bound immunoglobulin (Ig) have been removed, said Ig can be released using specific means for this purpose. Therefore, the nanoparticles of the invention can be used not only in methods for pre-treating samples in order to concentrate immunoglobulins in diagnostic applications where it is detected that a specific immunoglobulin, for example IgG, is at low levels (such as allergies, other autoimmune diseases and infectious diseases), but also in applications such as purifying Igs from culture media. After eluting the immunoglobulin bound to the nanoparticle of the invention, this purified immunoglobulin could also be used to evaluate its role in some types of allergic reactions (since, for example, the role of IgE is well established, but in some types of allergy, the role of IgG is still uncertain).

Likewise, there is also proposed a method for pre-treating samples and for diagnosing infections and/or autoimmune diseases in which the purification and/or concentration of the immunoglobulins involved in these clinical conditions is relevant.

In summary, the present invention, therefore, demonstrates that there is a particular pore size that allows optimal binding of protein G' or protein A to the mesoporous nanoparticles in a conformation that allows their immunoglobulin, preferably IgG, capture performance to be optimised.

Therefore, in one aspect, the present invention relates to a silica mesoporous nanoparticle comprising a covalently bound protein G' or protein A, wherein the mean pore size of the nanoparticle is equal to or greater than 10 nm in diameter (hereinafter, "nanoparticle of the invention").

In the present invention, "nanoparticle" is understood to mean a particle with dimensions on the nanometre scale, such as dimensions between 1,000 nm and 40 nm, between 750 nm and 40 nm, between 500 nm and 40 nm, between 250 nm and 40 nm, between 200 nm and 40 nm, between 150 nm and 40 nm, between 100 nm and 40 nm, between 50 nm and 40 nm.

The nanoparticle of the invention may have a spherical shape, although other shapes are not ruled out, such as, although in a non-limiting manner, an ellipsoid, a rod, a cone, a cube, a cuboid (for example, a rectangular box), a pyramid, and an irregular shape, etc. In certain cases, combinations of different nanoparticle shapes may be included. As previously mentioned, the nanoparticle may have a substantially spherical shape and, therefore, may have dimensions measured as a diameter of the sphere, such as an average diameter of between 1,000 nm and 40 nm, between 750 nm and 40 nm, between 500 nm and 40 nm, between 250 nm and 40 nm, between 200 nm and 40 nm, between 150 nm and 40 nm, between 100 nm and 40 nm, between 50 nm and 40 nm. The term "average" as used herein is intended to be the arithmetic mean. In some particular embodiments, a substantially spherical nanoparticle has an average diameter of 100 nm.

In a more particular embodiment, the nanoparticle of the invention is spherical and more preferably has a diameter of between 70 and 100 nm.

In the present invention, "silica mesoporous nanoparticle" is understood to mean a nanoparticle comprising silicon dioxide (silicon (IV) oxide). Nevertheless, other mesoporous nanoparticles that could also be used in the present invention are silicon or carbon mesoporous nanoparticles.

The nanoparticle of the invention is a silica mesoporous nanoparticle having one or more pores exposed on the surface of the nanoparticle, such that the accessible surface of the nanoparticle includes the outer surface of the nanoparticle and the inner surfaces of one or more pores within the nanoparticle. In a particular embodiment, the silica mesoporous material of the nanoparticle of the invention has a specific surface area of from 200 to 1,400 m²g⁻¹, preferably, from 400 to 1,200 m²g⁻¹, more preferably from 600 to 1,200 m²g⁻¹.

Methods for determining pore size are widely known in the state of the art. Examples of these methods include, but are not limited to, adsorption-desorption experiments, mercury porosimetry, SAS (small-angle scattering), NMR (nuclear magnetic resonance) and STM-AFM (scanning tunnelling and atomic force microscopies), among others. The average pore size is generally determined by means of BET-BJH calculation of N₂ desorption/adsorption isotherm data.

To date, it was unknown in the state of the art that there is a specific pore size, within the mesoporous range, in which, by binding protein G' or protein A, the immunoglobulin capture capacity of nanoparticles is much higher than with other pore sizes (or non-porous particles). The inventors of the present invention tested nanoparticles with 5.75, 8.53 and 12.22 nm pore diameter, and observed that the 5.75 and 8.53 nm nanoparticles performed the same compared to one another, but nanoparticles with a pore size around 12 nm performed significantly better. To that end, in a particular embodiment of the nanoparticle of the invention, the mean pore size of the nanoparticle is between 10 nm and 15 nm in diameter, preferably between 11 and 14 nm, preferably between 11 and 13 nm, preferably between 11 and 12.5 nm, preferably between 11.5 and 12.5 nm, preferably between 12 and 12.5 nm, more preferably the mean pore size of the nanoparticle is 12 nm in diameter.

In a particular embodiment of the nanoparticle of the invention, the pores are homogeneously distributed over the nanoparticle, in other words, unlike other nanoparticles in the state of the art where the core is magnetic and compact (non-porous) and the silica mesoporous part is only in the outer shell, in the nanoparticles of the present invention the pores occupy the entire volume of the nanoparticle, not just the shell.

The nanoparticle of the invention comprises a covalently bound protein G' or protein A. In the present invention, "protein G'" is understood to mean a protein G devoid of the albumin recognition region(s). In a particular embodiment, protein G' comprises, or consists of, an amino acid sequence with a sequence identity of at least 60%, 65%, 70%, 75%, 80%, 85%, 90%, 91%, 92%, 93%, 94%, 95%, 96%, 97%, 98% or 99% with sequence SEQ ID NO: 1. In another more particular embodiment, protein G' comprises or consists of an amino acid sequence with a sequence identity of 100% with SEQ ID NO: 1. The present invention also encompasses recombinant proteins G'.

SEQ ID NO: 1
MKGETTTEAVDAATAEKVFKQYANDNGVDGEWTYDDATKTFTVTEKPEVIDASELTP
AVTTYKLVINGKTLKGETTTEAVDAATAEKVFKQYANDNGVDGEWTYDDATKTFTVTE
KPEVIDASELTPAVTTYKLVINGKTLKGETTTKAVDAETAEKAFKQYANDNGVDGVWT
YDDATKTFTVTE

In an even more preferred embodiment, the Protein G' used in the present invention is that marketed by Sigma Aldrich (Reference 08062).

In the present invention, "protein A" is understood to mean a protein that is originally found in the bacterial wall of *Staphylococcus aureus* and has immunoglobulin, especially IgG, recognition capacity. In a particular embodiment, protein A comprises or consists of an amino acid sequence with a sequence identity of at least 60%, 65%, 70%, 75%, 80%, 85%, 90%, 91%, 92%, 93%, 94%, 95%, 96%, 97%, 98% or 99% with sequence SEQ ID NO: 2. In another more particular embodiment, protein A comprises, or consists of, an amino acid sequence with a sequence identity of 100% with SEQ ID NO: 2. The present invention also encompasses recombinant proteins A.

The silica mesoporous nanoparticle of the invention is covalently bound to either protein G' or protein A. The terms "anchored" and "bound" are equivalent and may be used interchangeably throughout the present description. In the present invention, "covalently bound" is understood to mean the chemical bond in which the atoms involved share electrons, in other words, it is not an electrostatic interaction but rather a chemically stable bond such that it is unlikely for the protein to be lost from the surface of the nanoparticle as a result of non-specific interactions with other molecules or due to changes in the conditions of the medium during processing of the sample in which they are found. An example of covalent anchoring or binding includes, but is not limited to, an amide-type bond, which is achieved by means of carbodiimide conjugation chemistry. Other types of reactions that could be used are: thiol-maleimide reaction, azide-alkyne cycloaddition reaction. Any of them can be used in the context of the present invention. Nevertheless, in a particular embodiment of the nanoparticle of the invention, covalent binding between the nanoparticle and protein G' or protein A occurs by means of carbodiimide conjugation chemistry.

In the present invention, "carbodiimide conjugation chemistry" is understood to mean the activation of a carboxylic acid using a carbodiimide, such that a primary amine can subsequently react with the activated form of the acid, giving rise to the formation of a stable amide bond in physiological medium.

As is known by a person skilled in the art, both the surface of the nanoparticle and the surface of the pores can be functionalised. In the present invention, a surface is understood to be "functionalised" when said surface has been chemically modified with functional groups. Depending on the type of reaction chosen to bind protein G' or protein A, the surface will be functionalised with one functional group or another. For example, in carbodiimide chemistry, the reaction takes place between a carboxylic acid and an amine, so the nanoparticle must be functionalised with one or the other. Since a protein (which also has both carboxylic acids and amines) is being bound, it is preferable to have the carboxylic acid in the nanoparticle and to use the amines in the protein to prevent unwanted secondary reactions that would occur if it were done the other way around. If another type of reaction is selected, functionalisation should be as follows, for example: for a thiol-maleimide reaction, the particle should be functionalised with maleimide groups (and the protein should have thiol groups), for azide-alkyne cycloaddition binding, the particle should be functionalised with an azide or alkyne group (and the protein should be modified with the other one, since these groups are not natively present in proteins).

In a particular embodiment, the surface of the nanoparticle and pores is functionalised with 3-aminopropyltriethoxysilane (APTES). Functionalisation with APTES is done to arrange amine groups on the surface of the nanoparticles, which is an intermediate step to arrive at carboxylic acid-functionalised nanoparticles. This functional group, carboxylic acid, is necessary for binding protein G' or protein A.

Methods for obtaining the nanoparticle of the invention are widely known in the prior art, for example, the method described in Durfee, P.N. et al. 2016, ACS Nano 10: 8325-8345. Briefly, the method for obtaining the nanoparticle of the invention preferably comprises a first step a) of synthesising the silica mesoporous nanoparticles by means of any method known in the art, a step b) of extracting the surfactant, a step c) of functionalising the nanoparticles, preferably by means of amination, more preferably with 3-aminopropyltriethoxysilane (APTES) in toluene, a step d) of carboxylating the nanoparticles, preferably with succinic anhydride in tetrahydrofuran (THF), and a step e) of binding protein G' or protein A by means of carbodiimide chemistry.

Therefore, in another aspect, the present invention relates to a method for producing the nanoparticle of the invention, wherein said method comprises:
a. condensing tetraethyl orthosilicate (TEOS) in a two-phase system consisting of a water phase and an organic phase, wherein the aqueous phase comprises a mixture of cetyltrimethylammonium chloride (CTAC), triethanolamine and deionised water and the organic phase comprises in a mixture of cyclohexane with TEOS where the concentration of TEOS is between 4% and 6%, preferably 5%, more preferably this step (synthesis reaction of the starting nanoparticle) is carried out at 50°C for 24h,
b. extracting the CTAC surfactant and washing, preferably where the extraction of the surfactant is carried out by means of ion exchange with an ethanolic solution of ammonium nitrate (10 mg/ml) at reflux for 1 hour, followed by a second reflux for 2 hours in 12 mM HCI ethanolic solution,
c. functionalising the surface of the nanoparticle obtained in (b) and its pores with 3-aminopropyltriethoxysilane (APTES) in toluene, this reaction preferably being carried out in an inert atmosphere (using nitrogen), at reflux for 24 hours, using 0.5 µL APTES/mg SMN (silica mesoporous nanoparticle),
d. converting the amino groups added in step (c) into carboxylic acids by means of adding succinic anhydride, this reaction preferably being carried out in the presence of tetrahydrofuran (THF), under inert atmosphere and at room temperature for 24 hours, and
e. covalently binding protein G' or protein A by means of carbodiimide conjugation chemistry, the carboxylic acids on the surface of the particles preferably being activated by dispersing the nanoparticles in buffer with 2-morpholinoethanesulphonic acid (MES) with N-hydroxysuccinimide (NHS) and N-(3-dimethylaminopropyl)-N'-ethylcarbodiimide hydrochloride (EDC); after stirring at room temperature for 1 hour, the activated nanoparticles are collected, most preferably by centrifugation, and redispersed in phosphate buffered saline (PBS) with protein G' or protein A in solution; after being stirred at room temperature for 24 hours, particles with bound protein G' or protein A are washed with PBS and redispersed again in PBS.

Another aspect relates to a silica mesoporous nanoparticle comprising a covalently bound protein G' or protein A, wherein the mean pore size of the nanoparticle is equal to or greater than 10 nm in diameter, preferably between 10 nm and 15 nm in diameter, more preferably 12 nm in diameter, obtained by the above method.

In another aspect, the present invention relates to a composition comprising the nanoparticle of the invention. This composition of the invention may further comprise a (macro)porous matrix in which the nanoparticles of the invention are immobilised, where said matrix may be, for example, but not limited to, an affinity column.

In another aspect, the present invention relates to the *in vitro* use of the nanoparticle of the invention or of the composition of the invention for binding, extracting (or eliminating), isolating, purifying or concentrating immunoglobulins (preferably IgG) or a functionally equivalent fragment thereof, from a sample.

In a preferred embodiment of this *in vitro* use of the invention, the immunoglobulin is immunoglobulin G when the nanoparticle comprises bound protein G', or immunoglobulin A, D, E, M and/or G (except IgG3) when the nanoparticle comprises bound protein A. Likewise, the functionally equivalent fragment of an immunoglobulin is the Fab, F(ab')2 or Fc fragment when the nanoparticle comprises bound G' or protein A, or the scFv fragment when the nanoparticle comprises bound protein A.

In a particular embodiment, binding, extracting, isolating, purifying or concentrating immunoglobulins is carried out by means of affinity chromatography.

In the present invention, "sample" is understood to mean a representative part of a whole. In a particular embodiment, the sample is a biological sample isolated from a subject or a cell culture medium. In the present invention, the expression "biological sample isolated from a subject" refers to any fluid biological material that can be obtained from the individual, such as blood, serum, saliva, cerebrospinal fluid, peritoneal fluid, semen, sputum, tears, mucus, sweat, milk, brain extracts and the like. In a particular embodiment, the biological sample isolated from a subject is blood or serum. The term "isolated" implies that the biological sample has been separated or extracted from the human or animal body and optionally also from other naturally occurring components. Techniques for obtaining samples, particularly biological samples from a subject, are widely known in the prior art and any of them can be used in putting the present invention into practice.

In the present invention the term "subject" is equivalent to the term "individual", the two terms can therefore be used interchangeably throughout this description. "Subject" is understood to mean any animal belonging to any species. Nevertheless, in a particular embodiment, the subject is a mammal, preferably, a primate, more preferably, a human of any ethnic group, sex or age.

As discussed above, the advantage of the nanoparticle of the invention is that its immunoglobulin loading capacity is much higher than that of other nanoparticles having a similar composition but a different pore size. The reason for this technical effect is, as stated, the existence of an optimal pore size that allows, upon binding protein G' or A, that the immunoglobulin capture capacity is much higher.

To that end, the nanoparticle of the invention is proposed for use for isolating, purifying, concentrating, eliminating or detecting immunoglobulins, such as IgG, or a functionally equivalent part thereof, such as fragment antigen-binding or Fab, F(ab')2 fragments, or single-chain variable fragments or scFv. In the present invention, "immunoglobulins" is understood to mean any of the five isotypes corresponding to IgA, IgD, IgE, IgG and IgM. "IgG" is understood to mean one of the five isotypes of humoral antibodies produced by the body. There are four variants or subclasses of IgG designated by numbers: IgG1 to IgG 4, and any of them can be detected/isolated by means of using the nanoparticle of the invention, except IgG3 when the protein bound to the nanoparticle is protein A.

In another aspect, the present invention relates to an *in vitro* method for isolating, purifying or concentrating an immunoglobulin, preferably an IgG, or a functionally equivalent fragment of an immunoglobulin, from a sample, hereinafter referred to as the "method for isolating or purifying of the invention", which comprises:
(i) contacting the sample with the nanoparticle of the invention or with the composition of the invention under suitable conditions to allow the binding of immunoglobulins, preferably IgG, or a functionally equivalent fragment thereof present in the sample, to the nanoparticle, and
(ii) isolating immunoglobulins, preferably IgGs, or a functionally equivalent fragment thereof, bound to the nanoparticle obtained after step (i).

The sample referred to in this method of the invention may be, but is not limited to, an isolated biological sample or cell culture medium.

In this method of the invention, the immunoglobulin is immunoglobulin G when the nanoparticle comprises bound protein G', or immunoglobulin A, D, E, M and/or G (except IgG3) when the nanoparticle comprises bound protein A.

Suitable conditions to allow the binding of the immunoglobulins present in the sample to the nanoparticle of the invention are, preferably, stirring at room temperature for 30 to 120 minutes, more preferably for 60 minutes. In this sense, for example, the inventors have demonstrated an IgG elimination capacity greater than 90% in human serum by incubating 50 microlitres of serum with 1 mg of the nanoparticles described in the present invention for 60 minutes (with stirring) at room temperature.

The expressions "IgG", "functionally equivalent fragment thereof", "sample" and "subject" have been defined above in previous inventive aspects, as well as their particular embodiments, and are applicable to the present inventive aspect. Therefore, in a particular embodiment of the method of the invention, the sample is a biological sample, more particularly, a biological sample isolated from a subject. In another particular embodiment of the method for isolating of the invention, the subject is a mammal, preferably, a primate, more preferably, a human of any ethnic group, sex or age. Preferably, the biological isolate is a blood or serum sample.

In another aspect, the present invention relates to an *in vitro* method for pre-treating a biological sample isolated from a subject in order to subsequently diagnose an allergy, hereinafter the "first method for pre-treating of samples of the invention", which comprises:
(i) contacting a biological sample isolated from said subject with the nanoparticle or with the composition of the invention under suitable conditions to allow the binding of IgGs, or a functionally equivalent fragment thereof present in the sample, to the nanoparticle, and
(ii) removing or extracting the IgGs bound to the nanoparticle.

In another aspect, the present invention relates to an *in vitro* method for diagnosing an allergy, in a subject, hereinafter referred to as the "method for diagnosing an allergy of the invention", comprising steps (i) and (ii) of the method described in the previous paragraph and additionally step (iii):
(i) contacting a biological sample isolated from said subject with the nanoparticle or with the composition of the invention under suitable conditions to allow the binding of IgGs, or a functionally equivalent fragment thereof present in the sample, to the nanoparticle,
(ii) removing or extracting the IgGs bound to the nanoparticle, and
(iii) detecting levels of IgE in the biological sample obtained after the elimination of step (ii), wherein high levels of IgE with respect to a reference value are indicative that the subject suffers from an allergy.

Another aspect of the invention relates to an *in vitro* method for pre-treating a biological sample isolated from a subject in order to subsequently diagnose an autoimmune disease or infection, hereinafter referred to as the "second method for pre-treating samples of the invention", which comprises:
(i) contacting a biological sample isolated from said subject with the nanoparticle or with the composition of the invention under suitable conditions to allow the binding of the immunoglobulins, preferably IgGs, or a functionally equivalent fragment thereof present in the sample, to the nanoparticle, and
(ii) purifying and/or concentrating the immunoglobulins, preferably IgGs, or a functionally equivalent fragment thereof, bound to the nanoparticle obtained after step (i).

Another aspect of the invention relates to an *in vitro* method for diagnosing an autoimmune disease or infection in a subject which comprises steps (i) and (ii) of the method described in the previous paragraph and additionally step (iii):
(i) contacting a biological sample isolated from said subject with the nanoparticle or with the composition of the invention under suitable conditions to allow the binding of the immunoglobulins, preferably IgGs, or a functionally equivalent fragment thereof present in the sample, to the nanoparticle,
(ii) purifying immunoglobulins, preferably IgGs, or a functionally equivalent fragment thereof, bound to the nanoparticle obtained after step (i), and
(iii) quantifying the immunoglobulins, preferably IgGs, or a functionally equivalent fragment thereof, purified in step (ii),
wherein high levels of immunoglobulins, preferably IgGs, with respect to a reference value are indicative that the subject suffers from an autoimmune disease or an infection.

The terms "biological sample" and "subject" have been defined above. Said definitions are applicable to all the methods of the present invention.

As it is used in the present description, the term "diagnose" refers to the identification of the nature of an ailment, disease or other problem by means of examining symptoms, parameters or biomarkers in a subject. In the context of the present invention, the parameter required for the diagnosis is the presence of IgE, and the condition to be diagnosed is an allergy; or the presence of IgG and the condition to be diagnosed is an autoimmune disease or infection.

In the present invention, "allergy" is understood to mean all respiratory, nervous or eruptive disorders that occur in the immune system due to extreme sensitivity of the body to certain substances to which it has been exposed, and which under normal conditions do not cause such disorders. Therefore, examples of allergies include, but are not limited to, allergies to drugs, food, fungi, pollen (grasses, conifers, olive allergens, etc.), hair from animals (dogs, cats, etc.,), arthropods (mites, etc.), chemical compounds (insecticides, cleaning products, fertilizers, etc.) or the venom of insects such as hymenoptera (for example, bees, bumblebees or wasps).

IgE is one of the most important biomarkers in the diagnosis of allergic reactions, since it is an antibody produced by the immune system in response to an internal or external factor or agent that the body perceives as a threat, such as an allergen or a parasite. IgE is one of five types of immunoglobulins (A, G, M D and E). Normally, its blood concentration is very low, and its detection may be masked due to the presence of IgG, the levels of which are much higher. Therefore, in the first step of the method for diagnosing an allergy of the invention, said method comprises eliminating or extracting IgG in a biological sample isolated from said subject by means of using the nanoparticle of the invention. Once the IgG has been eliminated from the sample, IgE is detected. Techniques for detecting IgE are widely known in the state of the art, and any of them can be used in the diagnostic method of the invention.

Once levels of IgE have been quantified, these are compared with a reference value, and if levels of IgE are higher than the reference levels, then it can be concluded that the subject suffers from an allergy.

In the present invention "reference value" is understood to mean those levels of IgE in a biological sample isolated from a subject who does not suffer from an allergy, or the mean levels of IgE in a set of subjects who do not suffer from an allergy.

In the present invention "reference value" is also understood to mean levels of IgG, IgM, IgD or IgA, preferably IgG, in a biological sample isolated from a subject who does not suffer from infections or autoimmune diseases, or those values derived from the mean levels of said immunoglobulins in a set of subjects who do not have said clinical conditions.

In summary, the present invention relates to a silica mesoporous nanoparticle comprising a bound protein G' or protein A with a mean size of pore diameter as defined herein, to the composition comprising said nanoparticle, to the use thereof for capturing, purifying, eliminating and/or isolating immunoglobulins, preferably IgG, most preferably to facilitate the determination of IgE for diagnosing allergies, as well as to a method for purifying an immunoglobulin, preferably an IgG, and to a method for diagnosing infections or autoimmune diseases in a subject.

### DESCRIPTION OF THE FIGURES

**Figure 1****.** Characterisation of S-SMN, M-SMN and L-SMN by means of dynamic light scattering (top panel) and transmission electron microscopy (bottom panel).
**Figure 2****.** Characterisation of the materials prepared after each chemical modification step by means of dynamic light scattering (top left panel), Z-potential (bottom left panel) and infrared spectroscopy (right panel).

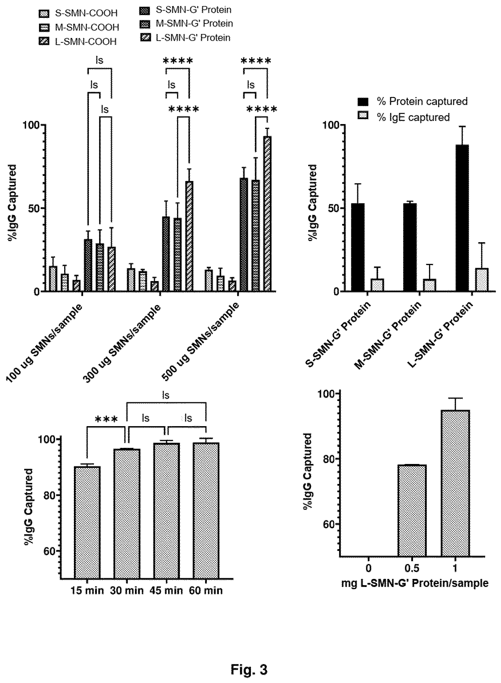

**Figure 3****.** Studies of IgG capture by the prepared materials. IgG capture in a 1 mg/ml IgG solution using different amounts of SMN-COOH and SMN-Protein G' (top left panel). Specificity studies of IgG capture using IgG + IgE mixtures, evaluating total protein capture by Nanodrop and IgE (FITC-labelled) capture by fluorometry (top right panel). IgG capture in a 1 mg/ml IgG solution at different L-SMN-Protein G' incubation times (lower left panel). IgG capture in a 10 mg/ml IgG solution using different amounts of L-SMN-Protein G' (lower right panel). Statistical analysis performed by means of two-way ANOVA for the upper left panel and by means of one-way ANOVA for the lower left panel, both using Graphpad Prism 9 software. "Is" p>0.05; ***p<0.001; ***p<0.0001.

### EXAMPLES

Next, the invention will be illustrated by means of tests carried out by the inventors, which demonstrate the effectiveness of the nanoparticle of the invention for capturing immunoglobulins.

### I. MATERIAL AND METHODS

### Preparation of protein G'-modified silica mesoporous nanoparticles.

The silica mesoporous nanoparticles that will serve as the basis for the system of the invention are prepared by a previously described two-phase method based on condensing tetraethyl orthosilicate (TEOS) in a water/cyclohexane two-phase system, using triethanolamine as the base and cetyltrimethylammonium chloride (CTAC) as the structure-directing surfactant agent (Durfee, P.N. et al. 2016, ACS Nano 10: 8325-8345). The aqueous phase is made up of a 24 ml mixture of a commercial aqueous solution of CTAC (25% w/v), 0.18 g triethanolamine and 36 ml deionised water. The organic phase consists of 20 ml of a mixture of cyclohexane with TEOS. The concentration of TEOS depends on the material to be prepared: 20% for S-SMN (small pore size silica mesoporous nanopores), 10% for M-SMN (medium pore size silica mesoporous nanopores) and 5% for L-SMN (large pore size silica mesoporous nanopores). The synthesis reaction is carried out at 50°C for 24 hours. Subsequently, the surfactant is extracted by ion exchange with an ethanolic solution of ammonium nitrate (10 mg/ml) at reflux for 1 hour, followed by a second reflux for 2 hours in 12 mM HCI ethanolic solution. Finally, the material is washed with ethanol 3 times to obtain the starting nanoparticles, without surfactant. The surface (both the outer surface of the particle and the exposed surface of the pores) is then functionalised with 3-aminopropyltriethoxysilane (APTES) in toluene (Paris, J. L. G., et al. 2020, Acta Biomater 101: 459-468). This reaction is carried out in an inert atmosphere (using nitrogen), at reflux for 24 hours, using 0.5 µL APTES/mg SMN (silica mesoporous nanoparticle). After washing the nanoparticles with toluene and ethanol, the added amino groups are converted into carboxylic acids (V. Lopez, V., et al. 2017, ACS Appl. Mater. Interfaces 9: 26697-26706). To do so, the aminated material is reacted with succinic anhydride (0.2 mg per mg SMN) in tetrahydrofuran (THF), under inert atmosphere and at room temperature for 24 hours.

Finally, and after washing the material with THF and ethanol, protein G' binding occurs by means of carbodiimide conjugation chemistry (Treerattrakoon, K., et al. 2017, Microchim. Minute 184: 1941-1950). To do so, carboxylic acids were activated on the surface of the particles by dispersing 5 mg of nanoparticles in 600 µL of buffer with 2-morpholinoethanesulphonic acid (MES, 50 mM; pH 5.5) with 1 mg N-hydroxysuccinimide (NHS) and 1 mg N-(3-dimethylaminopropyl)-N'-ethylcarbodiimide hydrochloride (EDC). After stirring at room temperature for 1 hour, activated nanoparticles were collected by centrifugation and redispersed in 500 µL of phosphate buffered saline (PBS) with 1 mg of protein G' in solution. After being stirred at room temperature for 24 hours, particles with bound protein G' were washed with PBS and redispersed again in PBS at a concentration of 10 mg/ml for further experiments.

### Characterisation of silica mesoporous nanoparticles.

To confirm the correct preparation and chemical modification of the nanoparticles, characterisation was performed by means of multiple techniques. Dynamic light scattering (DLS) and Z-potential measurements were performed with Malvern Zetasizer Nano ZS90 equipment, checking both particle size and surface charge. The instrument used is equipped with a "red laser" (λ = 300 nm) and DLS measurements were performed at a detection angle of 90°, while the Smoluchowski approximation was used for Z-potential measurements. For Fourier transform infrared spectroscopy (FTIR), Jasco 4100 FT/IR equipment equipped with an attenuated total reflectance (ATR) accessory was used. To check the morphology and different pore size of the nanoparticles, said nanoparticles were characterised by transmission electron microscopy (TEM) in Thermo Fisher Scientific Tecnai G2 20 Twin equipment, using 200 mesh size copper grids coated with a Formvar-Carbon film. Nitrogen adsorption (in Micromeritics ASAP 2020 equipment) and thermogravimetry (in Mettler Toledo TGA/DSC 1 equipment) measurements were performed at the Central Research Support Services (SCAI) of the University of Malaga (UMA).

### IgG removal experiments.

To evaluate the IgG removal capacity of the prepared nanoparticles, different amounts of nanoparticles (100, 300 or 500 µg) with or without bound protein G' were added to 300 µL of a solution of 1 mg/ml human IgG in PBS. After incubating at room temperature with agitation for 30 minutes, the samples were centrifuged and the amount of IgG still in the supernatant was quantified by means of spectrophotometry in Nanodrop 2000c equipment (Thermo Scientific). Subsequently, an analogous experiment was carried out by keeping the amount of nanoparticles constant (500 µg of particles for 300 µL of IgG solution) but evaluating the effect of incubation time (15, 30, 45 and 60 minutes) for removing IgG.

Finally, to evaluate the specificity of the capture by the system, immunoglobulin capture was evaluated in a mixture of IgG and IgE (which would be the analyte of interest in diagnosing an allergy). To be able to analyse IgE concentration in the solution with both immunoglobulins, fluorescent labelling of IgE was carried out using fluorescein isothiocyanate (FITC). IgE was labelled by incubating 25 µg of human IgE together with 2 µg of FITC in 100 µL of carbonate buffer (pH=10.2). After stirring at room temperature for one hour, labelled IgE was purified using an Amicon system (30kDa). Using this labelled IgE and unlabelled IgG, the mixture of IgG + IgE-FITC was prepared in PBS (1 mg/ml IgG, 1 µg/ml IgE-FITC). After incubating the nanoparticles with the IgG + IgE-FITC solution for half an hour at room temperature (500 µg of particles for 300 µL of the mixture of IgG + IgE-FITC), the samples were centrifuged and the supernatants were analysed by means of Nanodrop spectrophotometry to evaluate the total concentration of protein not captured by the nanoparticles. Subsequently, a 1:3 dilution of said supernatants with PBS was carried out to measure the IgE concentration using a fluorometer.

### II. RESULTS

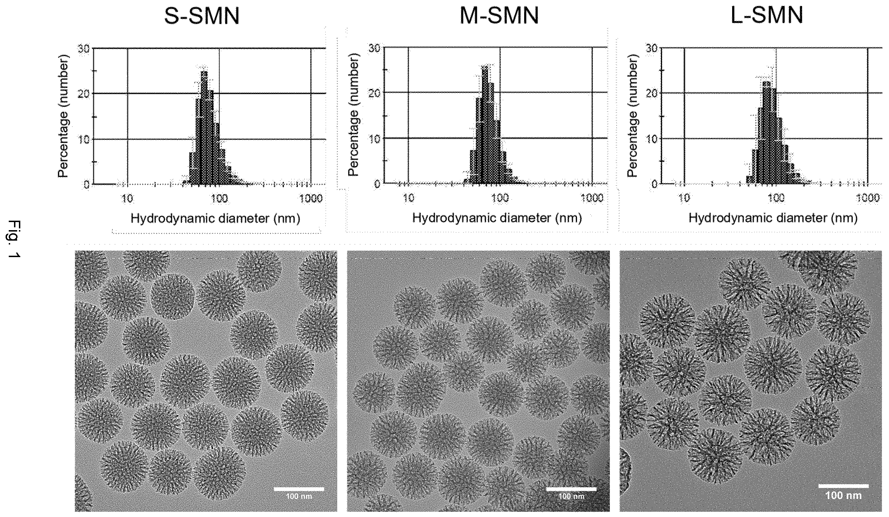

First, silica mesoporous nanoparticles (SMNs) with three different pore sizes were prepared: small (S-SMN), medium (M-SMN) and large (L-SMN). The correct preparation of the three materials was confirmed by means of dynamic light scattering (DLS), transmission electron microscopy (TEM) and nitrogen adsorption. DLS results showed the correct preparation of monodisperse particles with sizes in the range of 70-100 nm in diameter for the three types of particles prepared (Figure 1). The particle size observed by DLS was confirmed by means of TEM, and the presence of pores with the desired size order: S-SMN pore < M-SMN pore < L-SMN pore, was also observed (Figure 1). The pore size of each particle type was finally determined by nitrogen adsorption (Table 1), observing a pore size of 5.75 nm for S-SMN, 8.53 nm for M-SMN and 12.22 nm for L-SMN. In all cases, particles with a large surface area (> 400 m²/g) have been obtained.

**Table 1. Characterisation of S-SMN, M-SMN and L-SMN materials by means of nitrogen adsorption.**

| Sample | BET surface area (m²/g) | Pore volume (cm³/g) | Pore diameter (nm) |
|---|---|---|---|
| S-SMN | 417.5 | 0.56 | 5.75 |
| M-SMN | 455.3 | 0.8 | 8.53 |
| L-SMN | 679.5 | 1.41 | 12.22 |

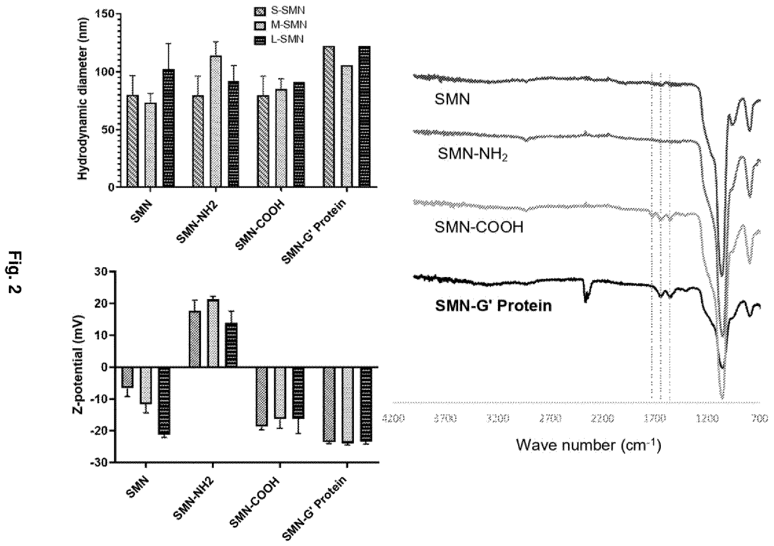

The prepared materials were then chemically modified in several steps: amination, carboxylation, protein G binding. The correct development of each step was confirmed by several characterisation techniques. DLS confirmed that no drastic change in particle size occurred in any step of the process, confirming that the particles did not undergo aggregation by any of the modifications made (Figure 2). Z-potential measurements (Figure 2) showed the change from negative values in the newly synthesised material to positive values after amination (SMN-NH₂), with a return to negative values after carboxylation (SMN-COOH), and also negative final values for materials with bound protein G' (SMN-Protein G'). Fourier transform infrared spectroscopy (FTIR) showed the characteristic bands of silica in all the prepared materials (700-1300 cm⁻¹). FTIR (Figure 2) also confirmed the presence of carboxylic acid groups in the carboxylated particles (band at 1720 cm⁻¹), as well as amide group signals confirming the covalent binding of the succinic anhydride used to the amino groups of the particles (amide I and amide II bands at 1650 and 1560 cm⁻¹, respectively). Finally, the disappearance of the carboxylic acid band and the increase in the relative intensity of the amide I and amide II signals confirms binding of the protein. The amount of protein G' bound to each material was determined by thermogravimetry (Table 2), showing a mass percentage of 6.88% for S-SMN, 7.89% for M-SMN and 8.94% for L-SMN. These data confirm the initial part of the hypothesis of the inventors by showing that the amount of protein G' bound to SMNs depends on the pore size of the nanoparticles.

**Table 2. Thermogravimetric characterisation of SMN-COOH and SMN-Protein G' materials for the three pore sizes prepared.**

| Sample | % Organic matter | | % Protein G' (mass) |
|---|---|---|---|
| | SMN-COOH | SMN-Protein G' | (Δ % organic matter) |
| S-SMN | 18.89 | 25.77 | 6.88 |
| M-SMN | 19.72 | 27.61 | 7.89 |
| L-SMN | 19.99 | 28.93 | 8.94 |

Once the desired nanoparticles have been obtained, their human IgG capture capacity was evaluated. To do so, different amounts of SMNs with bound protein G' were dispersed in a 1 mg/ml IgG solution. The same experiment was performed with carboxylated SMNs (without protein G') as a control. After half an hour incubation at room temperature, SMNs were removed by centrifugation and the amount of IgGs remaining in the supernatant was quantified. The results obtained (Figure 3) show that SMNs without protein G' have a very low non-specific IgG capture (less than 15%), and which does not appear to be dependent on particle concentration. Moreover, all SMNs with protein G' showed human IgG capture capacity, with an increase in the amount of IgG capture when increasing the particle concentration. From among the three materials prepared, particles with a larger pore size (L-SMN) exhibited a significantly higher IgG capture capacity than the other two materials, definitively confirming the initial hypothesis of the invention: by means of controlling the pore size of SMNs to which protein G' is bound, their IgG removal capacity may be optimised. It is important to note that the IgG capture capacity obtained with this material (450-800 µg of IgG per mg of particles) is much higher than that shown by other commercial systems or systems described in research (see Table 3 below), representing at least a 4-fold improvement with respect to the best performing commercial product (Magne^{™} Protein G Beads (Promega):

**Table 3. Capture capacity data from commercial systems (mostly with magnetic particles), according to their manufacturers.**

| Commercial systems | IgG capture capacity |
|---|---|
| SureBeads^{™} (Bio-Rad) | ≥6 µg IgG/mg particles |
| Pierce^{™} Protein G Magnetic Beads (ThermoFisher) | ≥60 µg IgG/mg particles |
| Absolute Mag^{™} Protein G Magnetic Particles, 100 nm (CD Creative Diagnostics) | >60 µg IgG/mg particles |
| Sera-Mag SpeedBeads Protein A/G (cytiva) | 55 - 85 µg IgG/mg particles |
| Magne^{™} Protein G Beads (Promega) | 125 µg human IgG/mg particles |

Additionally, the specificity of IgG capture in mixtures of IgG + IgE was evaluated, showing that the prepared nanoparticles are capable of capturing a high percentage of IgG while the removal of IgE (at a much lower concentration) was almost non-existent. With respect to the optimal incubation time, it was found that after 30 minutes, an increase in incubation time (to 45 or 60 minutes) did not lead to a significant increase in the amount of IgG captured.

Finally, the performance of the best prepared material (L-SMN with protein G') was evaluated in an IgG solution at a clinically relevant concentration (10 mg/ml), observing that with 1 mg of particles, more than 90% of the amount of IgG in 50 µL of this solution is removed after a single half-hour incubation. These conditions would be readily transferable to the clinical setting, showing the great potential of this material for pre-treating human serum for further use in an *in vitro* diagnosis of an allergy.

Furthermore, the preparation protocol described above in this example leads to SMNs with high inter-batch reproducibility, both in particle size and pore size, as well as in the various steps for functionalisation. Additionally, particle size and pore diameter distribution are also highly homogeneous, which is an important feature for developing *in vitro* techniques for quantifying biomarkers. These features also lead to good reproducibility for removing IgG, making the material suitable for being transferred to the clinical setting.

## Claims

1. A silica mesoporous nanoparticle comprising a covalently bound protein G' or protein A, wherein the mean pore size of the nanoparticle is equal to or greater than 10 nm in diameter.

2. The nanoparticle according to claim 1, wherein the mean pore size of the nanoparticle is between 10 nm and 15 nm in diameter, preferably 12 nm in diameter.

3. A composition comprising a nanoparticle according to any of claims 1 or 2.

4. A method for producing the nanoparticle according to any of claims 1 or 2, which comprises:
a. condensing tetraethyl orthosilicate (TEOS) in a two-phase system consisting of an aqueous phase and an organic phase, wherein the aqueous phase comprises a mixture of cetyltrimethylammonium chloride (CTAC), triethanolamine and deionised water and the organic phase comprises in a mixture of cyclohexane with TEOS where the concentration of TEOS is between 4% and 6%, preferably 5%,
b. extracting the CTAC surfactant and washing,
c. functionalising the surface of the nanoparticle obtained in (b) and its pores with 3-aminopropyltriethoxysilane (APTES) in toluene,
d. converting the amino groups added in step (c) into carboxylic acids by means of adding succinic anhydride, and
e. covalently binding protein G' or protein A by means of carbodiimide conjugation chemistry.

5. In vitro use of a nanoparticle according to any of claims 1 or 2 or of a composition according to claim 3, for binding, extracting, isolating, purifying or concentrating immunoglobulins, or functionally equivalent fragments thereof, from a sample.

6. Use according to claim 5, wherein the immunoglobulin is immunoglobulin G when the nanoparticle comprises bound protein G', or immunoglobulin A, D, E, M and/or G (except IgG3) when the nanoparticle comprises bound protein A.

7. The use according to any of claims 5 or 6, wherein the sample is a biological sample isolated from a subject or a cell culture medium.

8. The use according to claim 7, wherein the biological sample isolated from a subject is a blood or serum sample.

9. The use according to any of claims 7 or 8, wherein the subject is a human.

10. An in vitro method for pre-treating a biological sample isolated from a subject in order to subsequently diagnose an allergy which comprises:
(i) contacting a biological sample isolated from said subject with the nanoparticle according to any of claims 1 or 2 or with the composition according to claim 3 under suitable conditions to allow the binding of IgGs, or a functionally equivalent fragment thereof present in the sample, to the nanoparticle, and
(ii) eliminating the IgGs bound to the nanoparticle.

11. The in vitro method to diagnose an allergy in a subject which comprises steps (i) and (ii) of the method according to claim 10, and furthermore:
(iii) detecting levels of IgE in the biological sample obtained after the elimination of step (ii),
wherein high levels of IgE with respect to a reference value are indicative that the subject suffers from an allergy.

12. An in vitro method for pre-treating a biological sample isolated from a subject in order to subsequently diagnose an autoimmune disease or infection which comprises:
(i) contacting a biological sample isolated from said subject with the nanoparticle according to any of claims 1 or 2 or with the composition according to claim 3 under suitable conditions to allow the binding of immunoglobulins, preferably IgGs, or a functionally equivalent fragment thereof present in the sample, to the nanoparticle, and
(ii) purifying immunoglobulins, preferably IgGs, or a functionally equivalent fragment thereof, bound to the nanoparticle obtained after step (i).

13. The in vitro method to diagnose an autoimmune disease or infection in a subject which comprises steps (i) and (ii) of the method according to claim 12, and furthermore:
(iii) quantifying the immunoglobulins, preferably IgGs, or a functionally equivalent fragment thereof, purified in step (ii),
wherein high levels of immunoglobulins, preferably IgGs, with respect to a reference value are indicative that the subject suffers from an autoimmune disease or an infection.

14. An in vitro method for purifying or concentrating an immunoglobulin, preferably an IgG, or a functionally equivalent fragment thereof, from a sample which comprises:
(i) contacting the sample with a nanoparticle according to any of claims 1 or 2 or with the composition according to claim 3 under suitable conditions to allow the binding of immunoglobulins, preferably IgGs, or a functionally equivalent fragment thereof present in the sample, to the nanoparticle, and
(ii) isolating immunoglobulins, preferably IgGs, or a functionally equivalent fragment thereof, bound to the nanoparticle obtained after step (i).

15. The method according to claim 14, wherein the sample is an isolated biological sample or a cell culture medium.

16. The method according to any of claims 12 to 15, wherein the immunoglobulin is immunoglobulin G when the nanoparticle comprises bound protein G', or immunoglobulin A, D, E, M and/or G (except IgG3) when the nanoparticle comprises bound protein A.

17. The method according to any of claims 10 to 16, wherein the isolated biological sample is a blood or serum sample.

18. The method according to any of claims 10 to 17, wherein the subject from which the biological sample is taken is a human.
